# EUROPÄISCHE PATENTSCHRIFT

(11) **EP 2 288 920 B1**
(45) Veröffentlichungstag und Bekanntmachung des Hinweises auf die Patenterteilung: **24.10.2012**
(21) Anmeldenummer: 09768924.4
(22) Anmeldetag: 16.06.2009
(51) Int. Cl.: G01N 33/53

(54) **VERFAHREN ZUR BESTIMMUNG VON TRICHINELLA-INFEKTIONEN**
METHOD FOR THE DETERMINATION OF TRICHINELLA INFECTIONS
PROCÉDÉS DE DÉTERMINATION D'INFECTIONS À TRICHINELLA

(30) Priorität: 27.06.2008 DE 102008030129
(43) Veröffentlichungstag der Anmeldung: 02.03.2011
(73) Patentinhaber: Prionics AG, 8952 Schlieren (CH)
(72) Erfinder: RÄBER, Alex, CH-8046 Zürich (CH); HAUPT, Tina, CH-8455 Rüdlingen (CH); BUHOLZER, Patrik, CH-8409 Winterthur (CH)
(74) Vertreter: Emmel, Thomas
(86) Internationale Anmeldenummer: PCT/EP2009/004309
(87) Internationale Veröffentlichungsnummer: WO 2009/156075

(56) Entgegenhaltungen:
- US-A1- 2005 009 096
- US-A1- 2010 136 040
- REITEROVÁ K ET AL: "Trichinella spiralis-Outbreak in the Slovak Republic" 1. April 2007 (2007-04-01), INFECTION ; A JOURNAL OF INFECTIOUS DISEASE, URBAN & VOGEL, MU, PAGE(S) 89 - 93 , XP019511072 ISSN: 1439-0973 Zusammenfassung
- MIZOGUCHI ET AL: "Antineutrophil cytoplasmic antibodies in T-cell receptor alpha-deficient mice with chronic colitis" GASTROENTEROLOGY, ELSEVIER, PHILADELPHIA, PA, Bd. 113, Nr. 6, 1. Dezember 1997 (1997-12-01), Seiten 1828-1835, XP005111543 ISSN: 0016-5085
- PATTERSON R ET AL: "Studies of immunoglobulins, bentonite flocculation and IgE, IgG and IgM antibodies in serum from patients with trichinosis" 1. Juni 1975 (1975-06-01), AMERICAN JOURNAL OF MEDICINE,, PAGE(S) 787 - 793 , XP023221051 ISSN: 0002-9343 [gefunden am 1975-06-01] Tabelle II
- BIEN JUSTYNA: "[The usefulness of ELISA test for early serological detection of Trichinella spp. infection in pigs]" WIADOMOSCI PARAZYTOLOGICZNE 2007, Bd. 53, Nr. 2, 2007, Seiten 149-151, XP008109578 ISSN: 0043-5163
- MORAKOTE NIMIT ET AL: "Persistence of IgG, IgM, and IgE antibodies in human trichinosis" 1. September 1992 (1992-09-01), TROPICAL MEDICINE AND PARASITOLOGY, GEORG THIEME VERLAG, STUTTGART, DE, PAGE(S) 167 - 169 , XP008109573 ISSN: 0177-2392 Zusammenfassung; Tabelle 1

## Beschreibung

Die Erfindung bezieht sich auf ein Verfahren zur Bestimmung von Trichinella-Infektionen nach dem Oberbegriff des Anspruches 1

Trichinella spp. umfasst eine Gruppe von weltweit auftretenden Nematoden. Die in Europa vorherrschenden Spezies sind Trichinella spiralis, Trichinella britovi, Trichinella pseudospiralis und Trichinella nativa. Trichinella-Infektionen können bei unterschiedlichen Spezies einschließlich Menschen, Schweinen, Ratten, Bären, Pferden und Vögeln auftreten, um nur einige Beispiele zu nennen.

Es handelt sich bei Trichinellen um parasitische Nematoden, die einen definierten Lebenszyklus durchlaufen. Die Larven werden durch Verzehr von rohem oder ungenügend gekochtem Fleisch aufgenommen. Nach dem Verzehr werden sie durch die Magensäfte freigegeben, von wo aus sie in den Darmtrakt gelangen. Hier wachsen sie zu adulten Nematoden heran. Nach Paarung bringen die Weibchen bis zu 1500 neue Larven zur Welt, die über die Lymphe und den Blutkreislauf ins quergestreifte Muskelgewebe eindringen, insbesondere das Zwerchfell, Zunge, Auge und Kaumuskeln. Die Larven wachsen heran und bilden Larvenkomplexe, die im Laufe der Zeit kalzifizieren.

Trichinella-Infektionen stellen ein großes Problem bei der Tiererzeugung dar. Es muss sicher ausgeschlossen werden, dass Menschen nach Genuss von rohem oder nicht ausreichend gekochtem Fleisch von kranken Tieren an Trichinellosis erkranken. Menschliche Trichinellosis ist eine ernste Erkrankung, die erhebliche Leiden verursacht und in Extremfällen zum Tode führt.

Zum Zwecke der Nahrungsmittelsicherheit müssen z.B. daher alle Schweineschlachtkörper als Teil einer post-mortem Prüfung getestet werden. In der Regel werden die Schlachtkörper unmittelbar nach dem Schlachten weiterverarbeitet. Problematisch bei gängigen Tests ist, dass die Zerlegung in den dem Schlachthaus nachgeschalteten Zerlegebetrieben bereits zu einem gewissen Teil abgeschlossen ist, bevor die Ergebnisse der Trichinella-Tests zur Verfügung stehen. Dies stellt ein erhebliches logistisches Problem dar. Im Zweifelsfall müssen bei positiven Ergebnissen die zerlegten Schweineteile wieder aus dem Verarbeitungskreislauf herausselektiert und vernichtet werden.

Zugelassen sind zur Zeit Verfahren, in denen Trichinella direkt detektiert wird. Eine ältere nur noch während einer Übergangsfrist zugelassene Methode in Europa ist die sogenannte Trichinoskopie, bei der Gewebe zwischen zwei Glasplatten gepresst und dann mikroskopisch untersucht wird. Dieses Verfahren ist recht unempfindlich und darüber hinaus arbeits- und zeitintensiv. Außerdem können Trichinella-Arten, die sich nicht verkapseln, wie z. B. Trichinella-pseudospiralis nicht bzw. nur sehr schlecht entdeckt werden.

Eine weitere zugelassene Methode ist der sogenannte Verdautest, der sensitiver als die zuvor beschriebene direkte Detektion ist. Bei diesem Test wird das die Larve umgebende Muskelgewebe künstlich, mit Proteasen und Salzsäure verdaut und nach verscheidenden Sedimentationsschritten bleiben die Larven übrig. Anschließend kann die Anzahl der Larven mikroskopisch bestimmt werden. Das Detektionslimit liegt bei 3 bis 5 Larven pro Gramm Gewebe.

Wesentlich schneller und mit deutlich höherer Empfindlichkeit sind gattungsgemäße serologische Verfahren, bei denen zumeist auf indirektem Wege Antikörper gegen Trichinella spp. nachgewiesen werden. Solche serologischen Verfahren können zum Beispiel im Blutserum oder Fleischsaft durchgeführt werden. Übliche Verfahren haben ein Detektionslimit von 0,01 Larven auf ein Gramm Muskel-Gewebe.

Bei gattungsgemäßen serologischen Verfahren werden z.B. Serumproben aus dem zu untersuchenden Organismus mit Trichinella-Antigenen, z.B. dem exkretorischen/sekretorischen (E/S)-Antigen versetzt. Eventuelle aufgrund des Vorliegens einer Trichinella-Infektion im Serum enthaltene Antikörper binden an das Antigen. Die gebundenen Antikörper werden ihrerseits zum Beispiel mit markierten anti-Schwein-IgG-Antikörpern nachgewiesen. Gängige serologische Verfahren arbeiten z.B. mit immobilisierten Antigenen und weisen eventuell gebundene IgG-Antikörper nach. Spezielle weitere für serologische Verfahren geeignete Antigene sind z.B. in der WO 2007/090960 offenbart.

Reiterova K. et al.;(Infection 35(2); 2007; pp89-93) offenbart Bestimmung von Trichinella Infektion umfassend die Inkubation von Patientenserum mit auf ELISA-Platten gekoppeltem E/S Antigen und Bestimmung von IgG und IgM mittels entsprechender anti-Antikörper (Zusammenfassung).

Obwohl gattungsgemäße serologische Verfahren sehr empfindlich sind, sind sie im Moment noch nicht zur Fleischinspektion oder in Nahrungsmittel-Sicherheitsprogrammen zugelassen. Zwar sind serologische Verfahren schneller und empfindlicher als die direkten Tests wie z.B. der artifizielle Verdau, jedoch erlauben die vorliegenden EU-Regeln dennoch keine individuelle Testung von Schlachtkörpern mit serologischen Verfahren, da die bekannten serologischen Verfahren insbesondere in der Frühphase der Infektion falsch negative Resultate liefern können. Eine ähnliche Einstellung nimmt die internationale Trichinellosis-Kommission (ICT) ein. Auch hier wird bemängelt, dass das diagnostische Fenster für serologische Methoden nicht ausreicht und eventuell positive Tiere mit den zur Verfügung stehenden Methoden aufgrund des zu geringen Signals in der frühen Infektionsphase übersehen werden. Da aber auch in dieser Phase die Larven bereits im Muskel und damit infektiös sein können, müssen auch Tiere in der frühen Infektionsphase sicher erkannt werden.

Aufgabe der vorliegenden Erfindung ist es, ein sicheres serologisches Verfahren zur Diagnose von Trichinella-Infektionen bereitzustellen, mit dem sich sowohl frühe Infektionen als auch fortgeschrittene Trichinella-Infektionen sicher nachweisen lassen.

Gelöst wird die Aufgabe mit einem Verfahren gemäß Anspruch 1 Vorteilhafte Ausgestaltungen sind in den Unteransprüchen angegeben.

Das erfindungsgemäße Verfahren funktioniert im Wesentlichen wie oben angegeben. Eine Probe des zu untersuchenden Menschen oder Tieres, z.B. Serum oder Fleischsaft, wird mit bevorzugt immobilisierten Antigenen inkubiert. Eventuell in der Probe enthaltene Antikörper binden an die Antigene. Die Antikörper-Antigen-Komplexe werden dann mit markierten Antikörpern nachgewiesen. Es kann sich dabei insbesondere um ELISA-Verfahren handeln. Denkbar sind aber auch andere Verfahren mit anderen Markierungen, die z.B. optisch nachgewiesen werden können.

Wie oben bereits ausgeführt weisen übliche Trichinella-Tests die Antikörper-Antigen-Komplexe mit Antikörpern gegen IgG-Antikörper nach, da dies die üblicherweise im Infektionsverlauf zunehmend gebildeten persistenten Antikörper sind.

Erfindungsgemäß ist nun vorgesehen, dass außerdem gleichzeitig untersucht wird, ob IgM-Antikörper gebildet wurden. Dies kann auf einfache Weise durch Zugabe von Antikörpern gegen IgM-Antikörper erfolgen.

Erfindungsgemäß ist also vorgesehen, dass man die Probe auf das Vorliegen von IgM- und IgG-Antikörpern in Kombination untersucht. In der frühen Phase der Infektion werden vorrangig IgM-Antikörper gebildet, während der Gehalt an IgG-Antikörpern erst ab dem 15. Tag nach der Infektion beginnt. Der Gehalt an IgM-Antikörpern beginnt ungefähr zu diesem Zeitpunkt wieder abzunehmen.

Die relativ bald nach Infektion zu beobachtende Abnahme des Gehalts an IgM-Antikörpern ist auch ein wesentlicher Grund dafür, warum z. B. ELISA-Verfahren, mit denen IgM-Antikörper bestimmt werden, als nicht geeignet für die Diagnose von Trichinella-Infektionen betrachtet werden. Es wird hierzu stellvertretend auf eine am "Institut für Parasitologie" in Warschau, Polen, durchgeführte Doktorarbeit mit dem Titel "The usefulness of ELISA test for early serological detection of Trichinella spp. infection in pigs" verwiesen, die im Rahmen einer Disputation 2006 vorgestellt wurde. In der Doktorarbeit wurden Methoden einander gegenüber gestellt, in denen einerseits IgG-Antikörper und andererseits IgM-Antikörper detektiert wurden. Im Rahmen dieser Veröffentlichung wurde festgestellt, dass ELISA-Verfahren, mit denen IgG-Antikörper nachgewiesen werden, grundsätzlich zur Detektion von Trichinella-Infektionen geeignet sind. IgM-Antikörper wurden dagegen als nicht geeignete Marker der humoralen Reaktion von infizierten Tieren eingestuft. Ein wesentlicher Grund war, dass zumindest nach der in der Veröffentlichung vertretenen Meinung insbesondere Trichinella spiralis nicht nachgewiesen werden können. Ein weiterer Grund ist natürlich, dass die IgM-Konzentration relativ frühzeitig wieder abnimmt und das diagnostische Fenster daher nur frühe Infektionen abdecken kann.

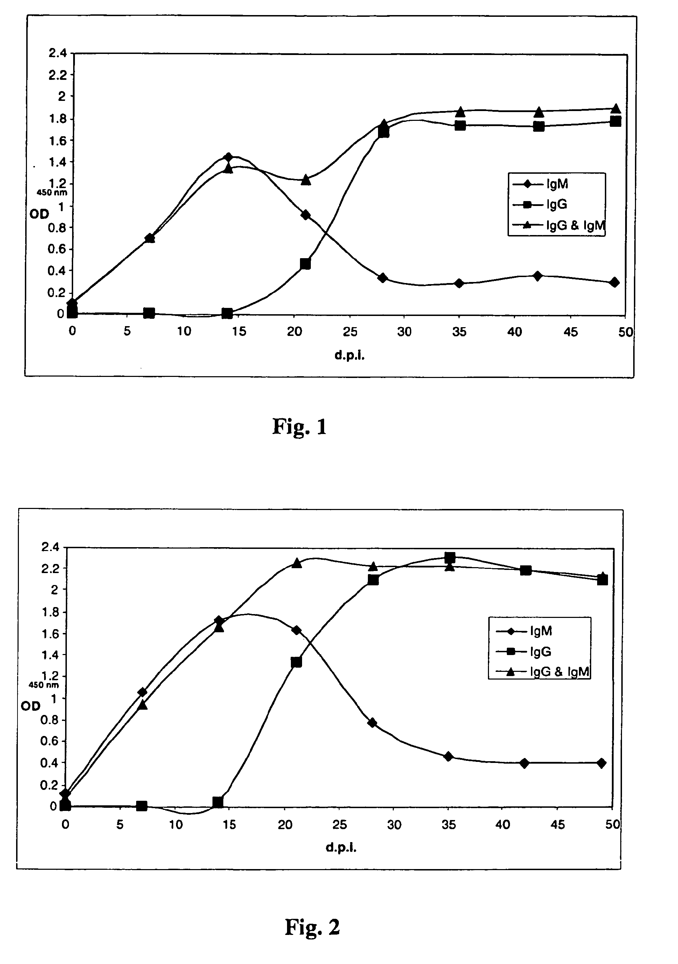

In eigenen Untersuchungen, die später anhand der Figuren 1 und 2 erläutert werden, haben die Anmelder herausgefunden, dass Antikörper der Klasse IgM auch bei Trichinella-spiralis-Infektionen auftreten und sich z. B. mit ELISA nachweisen lassen. Insoweit scheinen IgM-Antikörper entgegen der vertretenen Meinung doch grundsätzlich geeignete Marker zur Bestimmung von frühen Trichinella-Infektionen zu sein.

Ab Infektion mit Trichinellen liegt eine Immun-Antwort im Organismus vor, die sich mit dem erfindungsgemäßen Verfahren wenige Tage später detektieren lässt. Wie in Fig.1 oder 2 gezeigt, werden zunächst die IgM-Antikörper, im späteren Verlauf der Infektion IgM- und IgG-Antikörper und dann zunehmend nur IgG-Antikörper detektiert.

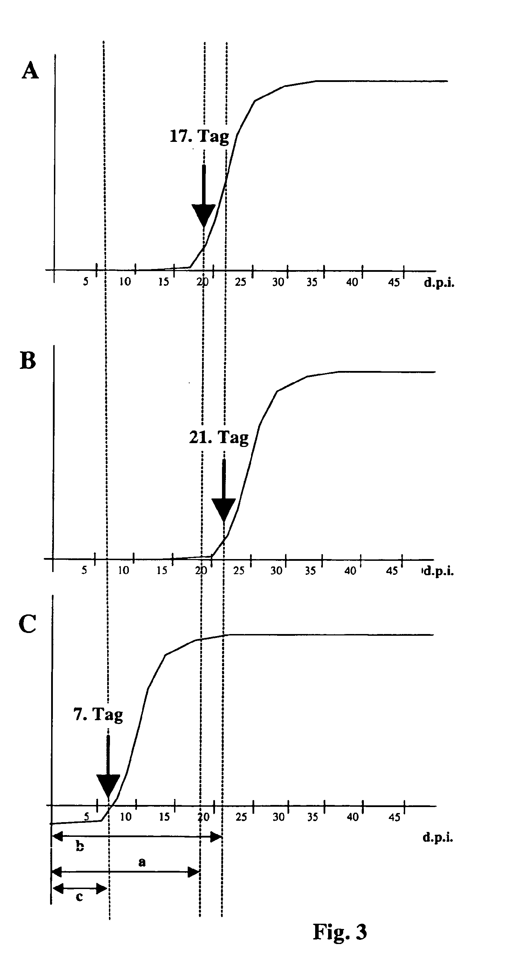

Das erfindungsgemäße Verfahren erkennt damit eine Infektion wesentlich früher als die bisher bekannten Methoden und verkleinert deshalb das diagnostische Fenster (siehe auch die unten diskutierte Fig. 3).

Mit dem von dem von den Anmeldern konzipierten Test, in dem erstmalig beide Antikörper-Antworten gleichzeitig detektiert werden, lassen sich überraschend die wesentlichen Nachteile bekannter serologischer Verfahren überwinden.

Im Rahmen der Offenbarung ist es möglich, generell das Vorliegen einer Antikörper-Antwort auf eine Trichinella-Infektion in einer Probe nachzuweisen, ohne die Antikörper einer Klasse zuzuordnen. Will man darüber hinaus auch noch Aufschluss darüber haben, ob es sich um ein frühes oder eher ein späteres Infektionsstadium handelt, dann ist es auch möglich, die in dem erfindungsgemäßen Verfahren eingesetzten Anti-Antikörper mit unterschiedlichen Markierungen zu versehen, je nachdem, ob sie zum Nachweis von IgG- oder IgM-Antikörpern gedacht sind.

Wie oben ausgeführt, werden in herkömmlichen und auch im erfindungsgemäßen serologischen Verfahren Trichinella-Antigene einer Probe zugesetzt. Bei den Antigenen kann es sich insbesondere um das E/S-Antigen handeln. Denkbar sind aber auch weitere immunogen wirkende Polypeptide, einzeln oder in Kombination, wie sie z. B. in der internationalen Patentanmeldung PCT/EP2007/005774 oder der WO 2007/090960 beschrieben werden.

Es können natürlich auch mehrere unterschiedliche Antigene, insbesondere Antigene, die zu unterschiedlichen Infektionsstadien auftreten, eingesetzt werden. Auf diese Weise bietet man eventuell enthaltenen Antikörpern unter Umständen bessere Bindungsmöglichkeiten, was ebenfalls zu einer Verbesserung der Aussage führt.

Um den Nachweis eventuell gebildeter Antigen-Antikörperkomplexe zu erleichtern, ist bevorzugt vorgesehen, dass die Antigene in immobilisierter Form eingesetzt werden. Denkbar ist z. B., die Wände von Mikrotiterplatten mit den Antigenen zu beschichten. Eine weitere Möglichkeit ist, die Antigene an Beads zu binden. Grundsätzlich sind alle Immobilisierungen geeignet, mit denen sich die Antikörper und eventuell daran gebundene Antikörper auf einfache Weise vom Rest der Probe trennen lassen.

Weiterhin ist erfindungsgemäß vorgesehen, dass die Anti-Antikörper eine mit üblichen Verfahren nachweisbare Markierung aufweisen. Es kann sich dabei z.B. um eine für einen ELISA-Nachweis geeignete Markierung handeln. Denkbar sind aber auch andere, z.B. optisch detektierbare Markierungen wie, z. B. Fluoreszenzmarker.

Bei den untersuchten Gewebeproben kann es sich insbesondere um Blut, Serum, Plasma, Fleischsaft handeln.

Neben einem Verfahren betrifft die Offenbarung auch eine diagnostische Zusammensetzung, die in dem Verfahren eingesetzt werden kann.

Eine solche Zusammensetzung enthält ein geeignetes, insbesondere immobilisiertes, Trichinella-Antigen sowie mit einer eine Detektierung erlaubenden Markierung versehene Anti-IgG- und Anti-IgM-Antikörper. Bevorzugt werden die Trichinella-Antigene und die Anti-Antikörper getrennt konfektioniert, so dass zunächst ein Kontakt zwischen den Antigenen und der Gewebeprobe hergestellt werden kann und nach einer ausreichenden Inkubationszeit dann die Zugabe der Anti-Antikörper erfolgen kann.

Bevorzugt wird das Trichinella-Antigen in immobilisierter Form vorgesehen. Denkbar ist z. B. eine Kopplung an Beads oder eine Beschichtung der Wände von Mikrotiterwells mit dem Antigen, um nur einige Beispiele zu nennen.

Im Wesentlichen dient die Immoblisierung des Antigens dazu, eine leichtere Trennung eventueller Antigen-Antikörper-Komplexe von der Probe zu ermöglichen. Diese Abtrennung erleichtert den spezifischen Nachweis der Komplexe.

Neben heterogenen Assays sind natürlich auch homogene Assays denkbar, d. h. Methoden, in denen das Antigen nicht immobilisiert vorliegt. Eine bevorzugte Methode wäre z.B. ein Fluoreszenz-Polarisationsassay. Die Durchführung einer solchen Methode ist dem Fachmann geläufig. Es soll daher hier nicht weiter darauf eingegangen werden.

Wie bereits zu dem Verfahren ausgeführt, enthält die Zusammensetzung als Antigen bevorzugt das sogenannte E/S-Antigen aus Trichinella bzw. immunogene Abschnitte dieses Antigens. Denkbar ist selbstverständlich auch der Einsatz weiterer Polypeptide aus Trichinella mit antigener Wirkung. Grundsätzlich können alle Peptide oder Polypeptide eingesetzt werden, die eine Serie von Aminosäuren aufweisen, die ein kontinuierliches oder diskontinuierliches Epitop ausbilden, das von Seren erkannt wird, die von mit Trichinella infizierten Schweinen stammen.

Denkbar ist selbstverständlich auch, mehrere unterschiedliche Antigene in der Zusammensetzung zu kombinieren. Die Antigene können als Gemisch vorliegen. Denkbar ist aber auch, sie zum Beispiel als Fusionsprotein miteinander gekoppelt vorzusehen.

Die Markierungen an den Anti-Antikörpern können schließlich so ausgebildet sein, dass sie auf einfache Weise einen optischen Nachweis ermöglichen. Bevorzugt werden jedoch ELISA-Verfahren eingesetzt. Selbstverständlich können die Anti-Antikörper daher auch mit entsprechenden Enzymen gekoppelt werden, die den erwünschten ELISA-Nachweis ermöglichen.

Die in Verbindung mit der Zusammensetzung beschriebenen Ausgestaltungen lassen sich, soweit dort noch nicht erwähnt, sinngemäß natürlich auch im Rahmen des Verfahrens umsetzen.

Im Folgenden soll die Erfindung anhand mehrerer Abbildungen im Detail erläutert werden.

Dabei zeigen:
**Fig. 1** und **Fig. 2** in schematischer Darstellung die Ergebnisse von ELISA-Verfahren bei der Bestimmung der Immunantwort von künstlich oral infizierten Schweinen mit 20.000 T. spiralis Larven und
**Fig. 3** (A)-(C) eine schematische Darstellung der mit unterschiedlichen Verfahren bei der Identifikation von Trichinella-Infektionen abgedeckten diagnostischen Fenster.

Die Figuren 1 und 2 zeigen jeweils die Ergebnisse von 3 ELISA-Verfahren. Dargestellt ist jeweils der Verlauf der IgG (■), der IgM (◆) sowie der kombinierten(▲) IgG- und IgM-Antwort auf eine Trichinella-Infektion zu unterschiedlichen Zeitpunkten.

Die Schweine wurden künstlich mit 20.000 T. spiralis Larven oral infiziert und Serumproben der infizierten Schweine zu unterschiedlichen Zeitpunkten untersucht. Fig. 1 und Fig. 2 zeigen Ergebnisse von Untersuchungen, die unter gleichen Bedingungen an Proben durchgeführt wurden, die von unterschiedlichen Schweinebeständen gewonnen wurden.

Proben wurden nach 0, 7, 14, 21, 28, 35, 42 und 49 auf die Infektion folgenden Tage (d. p. i. = Tage nach Infektion) entnommen. Es zeigte sich, dass die kombinierte Detektion der IgM- und IgG-Antwort bereits am 7. auf die Infektion folgenden Tag nachgewiesen werden konnte und dann über die gesamte Lebenszeit des Tieres erhalten blieb.

Dargestellt sind weiterhin die IgM-Reaktionen, die relativ frühzeitig nach der Infektion zu messen sind und die IgG-Antwort, die ab ca. dem 15. Tag detektierbar und dann relativ konstant über den Rest der Lebenszeit des Tieres nachweisbar ist.

Beide Figuren 1 und 2 zeigen ähnliche Verläufe der oben erwähnten Reaktionen.

Fig. 3 (A)-(C) zeigt schematisch die mit unterschiedlichen Verfahren [(A) = Verdautest; (B) = Herkömmlicher E/S ELISA und (C) = ELISA gemäss der Erfindung] bei der Identifikation von Trichinella-Infektionen abgedeckten diagnostischen Fenster.

Figur 3 (A) zeigt, dass der früheste Zeitpunkt, bei dem sich eine Trichinella-Infektion mit dem künstlichen Verdautest nachweisen lässt, der 17. Tag nach der Infektion ist.

Figur 3 (B) zeigt die Bedingungen bei der Identifikation von Trichinella-Infektionen in Schweinen unter Verwendung eines herkömmlichen E/S-ELISA-Verfahrens. Hier zeigt sich, dass mit einem herkömmlichen serologischen Verfahren ein Nachweis einer Immun-Antwort frühestens am 21. Tag nach Infektion möglich ist. Zu diesem Zeitpunkt können Larven bereits infektiös sein.

Figur 3 (C) zeigt nun die Bedingungen für das erfindungsgemäße Verfahren bei der Identifikation von Trichinella-infizierten Schweinen. Der früheste Zeitpunkt, bei dem sich in Proben aus Trichinella-infizierten Schweinen eine Antikörper-Antwort nachweisen lässt, ist der 7. Tag nach Infektion. Dies ist deutlich früher als bei den beiden unter (A) und (B) dargestellten Verfahren. Am 7. Tag sind die Larven weiterhin noch nicht infektiös. Es ist also davon auszugehen, dass das erfindungsgemäße Verfahren eine ausreichend sichere Bestimmung von Trichinella-Infektionen ermöglicht.

Der Vergleich der unterschiedlichen Nachweismethoden in (A), (B) und (C) ist mit gestrichelten Linien dargestellt. **b** bezeichnet das diagnostische Fenster für üblicherweise benutzte E/S-ELISA (B), a das diagnostische Fenster für den künstlichen Verdautest (A) während c das diagnostische Fenster für das erfindungsgemäße ELISA-Verfahren (C) zeigt. Auch aus dieser Darstellung wird deutlich, dass mit dem erfindungsgemäßen Verfahren infizierte Schweine zu einem signifikant früheren Zeitpunkt detektiert werden können. Das diagnostische Fenster wird dadurch deutlich enger, eine Detektion einer Trichinella Infektion ist sogar früher möglich als mit dem künstlichen Verdautest und macht ein derartiges Diagnoseverfahren geeignet für individuelle Fleischtestung im Schlachthof.

## Patentansprüche

1. Verfahren zur Bestimmung von Trichinella Infektionen bei einem Tier oder einem Menschen, umfassend die Inkubation einer dem Tier oder dem Menschen entnommenen Gewebeprobe mit Trichinella-Antigenen, die Aufbereitung der Gewebeprobe, die Zugabe von anti-Antikörpern zu der Gewebeprobe und die Überprüfung, ob eine Bindung der anti-Antikörper an eventuell in der Gewebeprobe enthaltene Antigen-Antikörper-Komplexe stattgefunden hat, **dadurch gekennzeichnet, dass** als anti-Antikörper gleichzeitig anti-IgG-Antikörper und anti-IgM-Antikörper zugesetzt werden.

2. Verfahren nach Anspruch 1, **dadurch gekennzeichnet, dass** als Antigen Trichinella E/S-Antigen eingesetzt wird.

3. Verfahren nach Anspruch 1 oder 2, **dadurch gekennzeichnet, dass** die eingesetzten Antigene in immobilisierter Form vorliegen.

4. Verfahren nach einem der vorhergehenden Ansprüche, **dadurch gekennzeichnet, dass** markierte anti-Antikörper eingesetzt werden.

5. Verfahren nach einem der vorhergehenden Ansprüche, **dadurch gekennzeichnet, dass** anti-IgG-Antikörper mit einer anderen Markierung versehen sind als anti-IgM-Antikörper.

6. Verfahren nach einem der vorhergehenden Ansprüche, **dadurch gekennzeichnet, dass** die Gewebeprobe Serum, Plasma oder Fleischsaft ist.

## Claims

1. A method for the determination of Trichinella infections in an animal or a human, comprising the incubation of a tissue sample, taken from the animal or the human, with Trichinella antigens, the processing of the tissue sample, the addition of anti-antibodies to the tissue sample, and testing whether a binding of the anti-antibodies to any antigen/anti-antibody complexes which may be present in the tissue sample has taken place, **characterized in that** anti-IgG antibodies and, simultaneously, anti-IgM antibodies are added as anti-antibodies.

2. The method as claimed in claim 1, **characterized in that** the antigen used is Trichinella E/S antigen.

3. The method as claimed in claim 1 or 2, **characterized in that** the antigens used are present in immobilized form.

4. The method as claimed in any of the preceding claims, **characterized in that** labeled anti-antibodies are used.

5. The method as claimed in any of the preceding claims, **characterized in that** anti-IgG antibodies are provided with a different label than anti-IgM antibodies.

6. The method as claimed in any of the preceding claims, **characterized in that** the tissue sample is serum, plasma or meat juice.

## Revendications

1. Procédé permettant de mettre en évidence une infection par Trichinella chez un animal ou un humain comprenant l'incubation d'un échantillon de tissu prélevé sur l'animal ou l'humain avec des antigènes Trichinella, la préparation de l'échantillon de tissu, l'adjonction d'anti-anticorps à l'échantillon de tissu et la vérification si une liaison des anti-anticorps avec des complexes antigène-anticorps éventuellement contenus dans l'échantillon de tissu a eu lieu, **caractérisé en ce que** l'on ajoute comme anti-anticorps simultanément des anticorps anti-IgG et des anticorps anti-IgM.

2. Procédé selon la revendication 1, **caractérisé en ce que** l'antigène utilisé est un E/S de Trichinella.

3. Procédé selon la revendication 1 ou 2, **caractérisé en ce que** les antigènes utilisés le sont sous forme immobilisée.

4. Procédé selon l'une des revendications précédentes **caractérisé en ce qu'**il met en oeuvre des anti-anticorps marqués.

5. Procédé selon l'une des revendications précédentes **caractérisé en ce que** les anticorps anti-IgG sont marqués différemment des anticorps anti-IgM.

6. Procédé selon l'une des revendications précédentes **caractérisé en ce que** l'échantillon de tissu est du sérum, du plasma ou du jus de viande.
